# EUROPÄISCHE PATENTSCHRIFT

(11) **EP 1 272 480 B1**
(45) Veröffentlichungstag und Bekanntmachung des Hinweises auf die Patenterteilung: **23.11.2005**
(21) Anmeldenummer: 01923629.8
(22) Anmeldetag: 05.03.2001
(51) Int. Cl.: C07D 307/60

(54) **VERFAHREN ZUR HERSTELLUNG VON SPIROCYCLISCHEN TETRONSÄUREDERIVATEN**
METHOD FOR THE PRODUCTION OF SPIROCYCLIC TETRONIC ACID DERIVATIVES
PROCEDE POUR PRODUIRE DES DERIVES D'ACIDE TETRONIQUE SPIROCYCLIQUES

(30) Priorität: 16.03.2000 DE 10012825
(43) Veröffentlichungstag der Anmeldung: 08.01.2003
(73) Patentinhaber: Bayer CropScience AG, 40789 Monheim (DE); BAYER CORPORATION, Pittsburgh, PA 15205 (US)
(72) Erfinder: FALBE, Volker, 42115 Wuppertal (DE); KULKARNI, Shekhar, V., Shawnee, KS 66216 (US)
(74) Vertreter: Krieg, Robert Alexander
(86) Internationale Anmeldenummer: PCT/EP2001/002440
(87) Internationale Veröffentlichungsnummer: WO 2001/068625

(56) Entgegenhaltungen:
- EP-A- 0 528 156
- DE-A- 4 337 853
- US-A- 5 585 504

## Beschreibung

Die vorliegende Erfindung betrifft ein neues Verfahren zur Herstellung bekannter spirocyclischer Tetronsäurederivate.

Bekannt ist die mehrstufige Synthese spirocyclischer Tetronsäurederivate (EP-A-528 156).

Es wurde gefunden, dass man eine Verbindung der Formel (Ia) 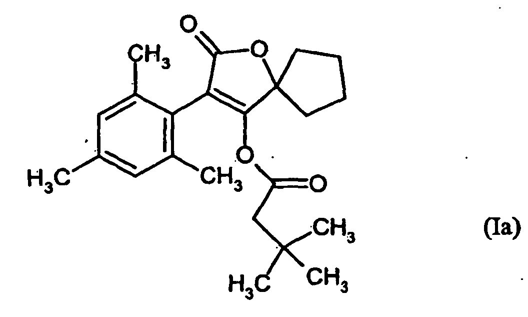 erhält, indem man Verbindung der Formel (IIa) 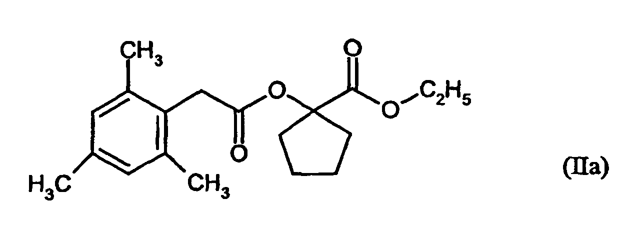 zunächst mit NaOH und auschließend mit der Verbindung der Formel (IIIa) 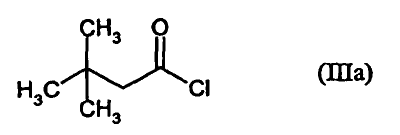 gegebenenfalls in Gegenwart eines Verdünnungsmittels umsetzt.

Aüßerdem wurde gefünden, dass man eine Verbindung der Formel (Ib) 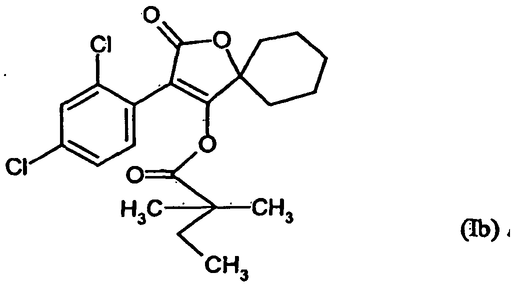 erhält, wenn man Verbindung der Formel (IIb) 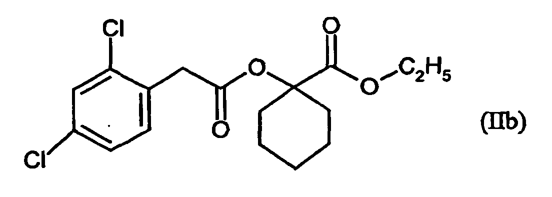 zunächst mit NaOH und auschließend mit der Verbindung der Formel (IIIb) 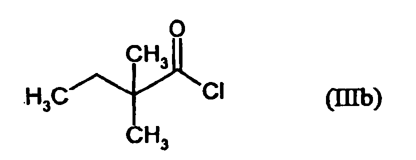 gegebenenfalls in Gegenwart eines Verdünnungsmittels umsetzt.

Überraschenderweise können nach dem erfindungsgemäßen Verfahren die o.g. Verbindungen auf einfachere Weise, in einem Eintopfverfahren, ohne Isolierung der Zwischenstufen, in höherer Reinheit und in besserer Ausbeute hergestellt werden.

Als Basen (Deprotonierungsmittel) für die Ringschlußreaktion können alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatälysatoren wie z.B. Triethylbenzylammoniumchlorid, Tetrabutylammoniumbromid, Adogen 464 (Methyltrialkyl (C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natriummethylat, Natrium-ethylat und Kalium-tert.-butylat oder auch tert.-Amine wie Diazabicyclooctan (DABCO), Diazabicycloundecan (DBU), Diazabicyclononen (DBN) und Hünig-Base einsetzbar.

Als Verdünnungsmittel für die Ringschlußreaktion können alle gegenüber der eingesetzten Base inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chlorbenzol und o-Dichlorbenzol, außerdem Ether, wie Diethylether, Methyl-tert.-butylether, tert.-Amylether, Tetrahydrofuran und Dioxan, darüberhinaus stark polare Solventien, wie N,N-Dimethylformamid, N,N Dimethylacetamid, N-Methylpyrrolidon, Dimethylsulfoxid und Sulfolan.

Nach erfolgter Ringschlußreaktion wird das Säurehalogenid zur Reaktionslösung gegeben.

Zum Abfangen von Chlorwasserstoffresten aus der Säurechloridherstellung können geringe Mengen üblicher Säureakzeptoren zugesetzt werden. Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethylanilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat.

Die Reaktionstemperaturen können bei dem erfindungsgemäßen Verfahren innerhalb eines größeren Bereiches variiert werden. Im allgemeinen arbeitet man bei Temperaturen zwischen -20°C und +200°C, vorzugsweise zwischen 0°C und 150°C.

Die Reaktion erfolgt im allgemeinen unter vermindertem Druck, vorzugsweise in einem Bereich von 50 - 500 mbar.

Bei der Durchführung des erfindungsgemäßen Verfahrens setzt man die Reaktionskomponenten der Formeln (IIa), (IIb), (IIIa) und (IIIb) und die deprotonierenden Basen im allgemeinen in etwa äquimolaren Mengen ein. Es ist jedoch auch möglich, die eine oder andere Komponente in einem größeren Überschuss (bis zu 5 Mol, vorzugsweise bis zu 2 Mol) zu verwenden.

Die Ausgangsstoffe der Formel (IIa) und (IIb) sind bekannt. Ihre Herstellung ist in EP-A-647 637 beschrieben. Die Carbonsäurehalogenide der Formel (IIIa) und (IIIb) sind ebenfalls bekannt. Sie sind kommerziell erhältlich oder können nach allgemein üblichen Verfahren der organischen Chemie hergestellt werden.

Die Herstellung der Verbindungen soll durch die folgenden Herstellungsbeispiele erläutert werden.

### Herstellungsbeispiele

### Beispiel 1

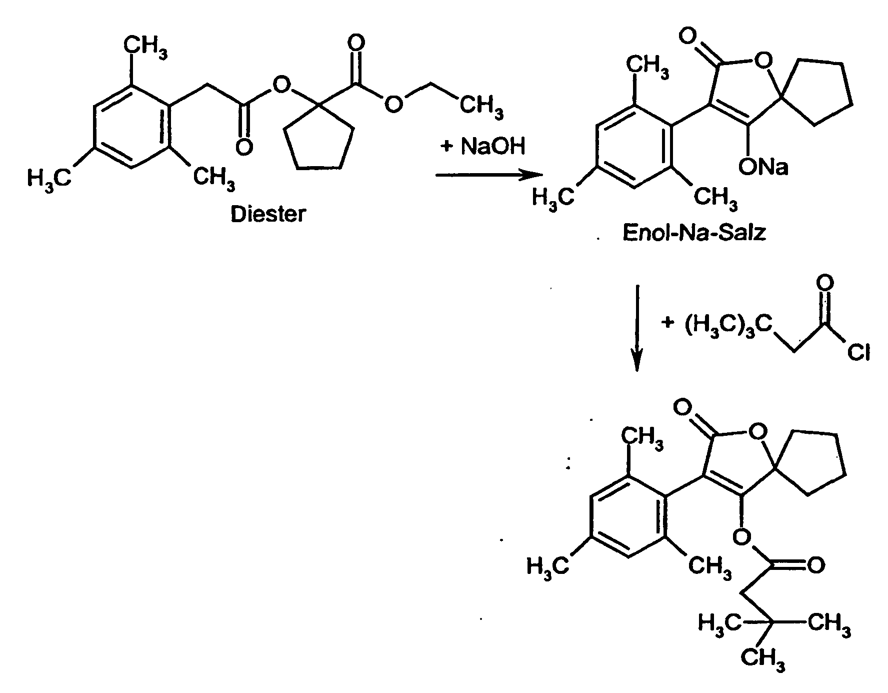

In einem Kolben werden 1,1 mol Natriumhydroxid in 500 ml N,N-Dimethylacetamid vorgelegt, ein Vakuum von 200 mbar angelegt und die Mischung erhitzt. Bei gleichzeitiger Destillatabnahme von ca. 50 ml/h wird dann eine Lösung von 1 mol Diester in ca. 225 ml N,N-Dimethylacetamid in 4 h zudosiert. Es wird 2 h bei weiterer Destillatabnahme von ca. 50 ml/h nachgerührt. Anschließend wird bei 200 mbar noch weitere ca. 70 g Destillat abgenommen. Die Menge an Destillationssumpf beträgt am Ende 688,5 g.

Der Umsatz zum Enol-Na-Salz wird wie folgt bestimmt:

1/10 des Destillationssumpfes wird bei Raumtemperatur unter Rühren in 150 ml 5 %ige Salzsäure gegeben. Das ausgefällte Enol wird abfiltriert, mit 100 ml Wasser ausgerührt, erneut filtriert und im Vakuum bei 50°C getrocknet. Es wird 22,4 g Enol mit einem Gehalt von 88,0 % (72,4 % d. Th.) erhalten; in der Mutterlauge (152,4 g) wird noch 1,57 % (8,8 % d. Th.), im Waschwasser (101,5 g) noch 0,43 % (1,6 % d. Th.) Enol nachgewiesen. Die Gesamtausbeute an Enol beträgt somit 82,8 % d. Th.

137,7 g des Sumpfes wird in einem Kolben zusammen mit ca. 15 mol-% Triethylamin (zum Abfangen von HCl-Resten aus der Säurechloridherstellung) in 130 ml Methylcyclohexan vorgelegt. Bei 25 bis 30°C wird 0,25 mol 3,3-Dimethylbuttersäurechlorid in 2 h zudosiert. Es wird 2 h bei 25 bis 30°C nachgerührt. Anschließend wird die Reaktionsmischung ohne Kühlung in eine Lösung von 8,4 g Natriumhydrogencarbonat in 320 ml Wasser dosiert. Das Gemisch wird auf 50°C aufgeheizt und 1 h bei dieser Temperatur gerührt. Nach Abtrennung der wässrigen Phase werden 80 ml Wasser zugegeben. Es wird 30 min bei 50°C gerührt und die wässrige Phase abgetrennt. Die organische Phase wird auf 35°C abgekühlt, mit Impfltristallen versetzt und 1 h bei 25°C gerührt. Anschließend wird langsam auf -10°C abgekühlt und 1 h bei dieser Temperatur gerührt.
Ausgefallenes Produkt wird filtriert und getrocknet: 51,7 g (68,4 % d. Th. bezogen auf eingesetzten Diester)

### Beispiel 2

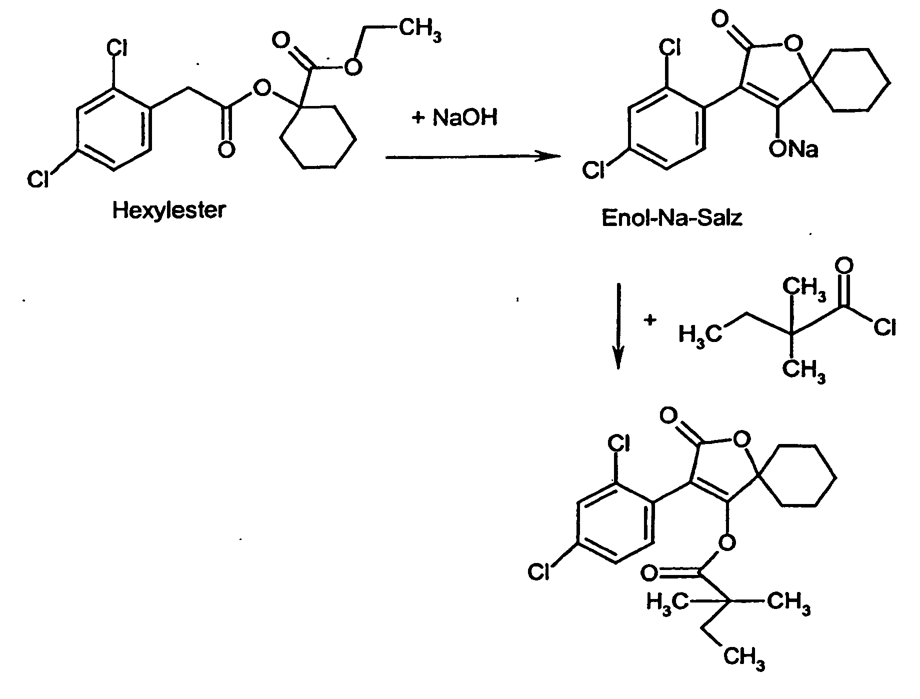

In einem Kolben werden 2,2 mol Natriumhydroxid in 1000 ml N,N-Dimethylacetamid vorgelegt, ein Vakuum von 200 mbar angelegt und die Mischung erhitzt. Bei gleichzeitiger Destillatabnahme von ca. 200 g/h wird dann eine Lösung von 2 mol Hexylester in ca. 1 150 ml N,N-Dimethylacetamid in 4 h zudosiert. Es wird 2 h bei weiterer Destillatabnahme von ca. 200 g/h nachgerührt. Anschließend wird bei 200 mbar noch weitere ca. 120 g Destillat abgenommen. Die Menge an Destillationssumpf beträgt am Ende 1 484 g.

Der Umsatz zum Enol-Na-Salz wird auf zwei Arten bestimmt:
1. Eine Probe des Sumpfes wird mittels HPLC untersucht. Dabei wird das Na-Salz als freies Enol bestimmt. Es wird ein Gehalt von 40,0 % Enol ermittelt; dies entspricht einer Ausbeute von 94,8 % d. Th. bezogen aufHexylester.
2. 1/20 des Destillationssumpfes wird bei Raumtemperatur unter Rühren in 150 ml 5 %ige Salzsäure gegeben. Das ausgefällte Enol wird abfiltriert, mit 100 ml Wasser ausgerührt, erneut filtriert und im Vakuum bei 50°C getrocknet. Es werden 32,4 g Enol mit einem Gehalt von 94,4 % erhalten; dies entspricht einer Ausbeute von 97,6 % d. Th. bezogen auf Hexylester.

148 g des Sumpfes wird in einem Kolben zusammen mit ca. 15 mol-% Triethylamin (zum Abfangen von HCl-Resten aus der Säurechloridherstellung) in 130 ml Methylcyclohexan vorgelegt. Bei 25 bis 30°C wird 0,25 mol 2,2-Dimethylbuttersäurechlorid in 2 h zudosiert. Es wird 2 h bei 25 bis 30°C nachgerührt. Anschließend wird die Reaktionsmischung ohne Kühlung in eine Lösung von 8,4 g Natriumhydrogencarbonat in 320 ml Wasser dosiert. Das Gemisch wird auf 50°C aufgeheizt und 1 h bei dieser Temperatur gerührt. Nach Abtrennung der wässrigen Phase werden 80 ml Wasser zugegeben. Es wird 30 min bei 50°C gerührt und die wässrige Phase abgetrennt. Die organische Phase wird auf 35°C abgekühlt, mit Impflaristallen versetzt und 1 h bei 25°C gerührt. Anschließend wird langsam auf-10°C abgekühlt und 1 h bei dieser Temperatur gerührt. Ausgefallenes Produkt wird filtriert und getrocknet: 73,7 g (89,1 % d. Th. bezogen auf eingesetzten Hexylester)

## Patentansprüche

1. Verfahren zur Herstellung der Verbindung der Formel (Ia) 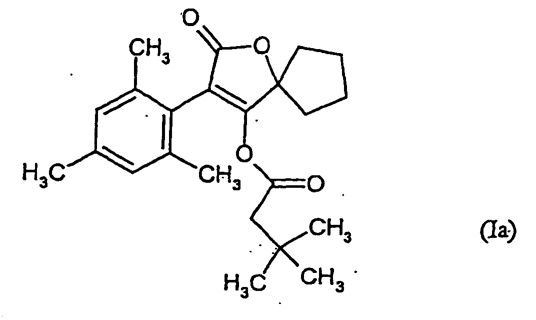 **dadurch gekennzeichnet, dass** man die Verbindung der Formel (IIa) 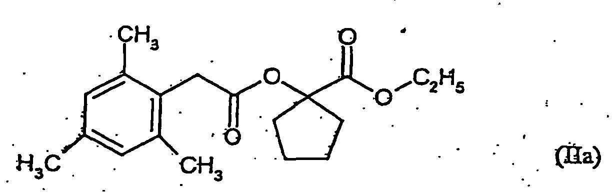 zunächst mit NaOH und anschließend mit der Verbindung der Formel 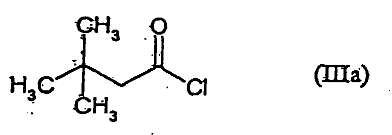 gegebenenfalls in Gegenwart eines Verdünnungsmittels umsetzt.

2. Verfahren zur Herstellung der Verbindung der Formel (Ib) 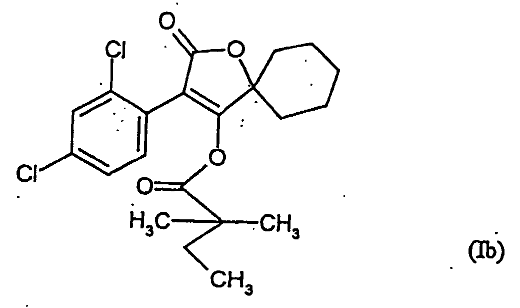 **dadurch gekennzeichnet, dass** man die Verbindung der Formel (IIb) 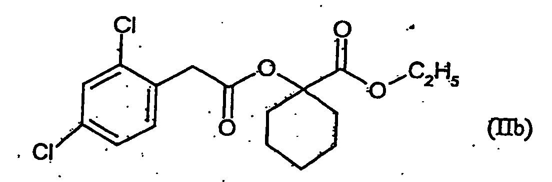 zunächst mit NaOH und anschließend mit der Verbindung der Formel (IIIb) 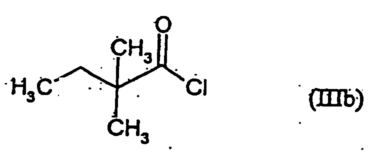 gegebenenfalls in Gegenwart eines Verdünnungsmittels umsetzt.

## Claims

1. Process for preparing the compound of the formula (Ia) 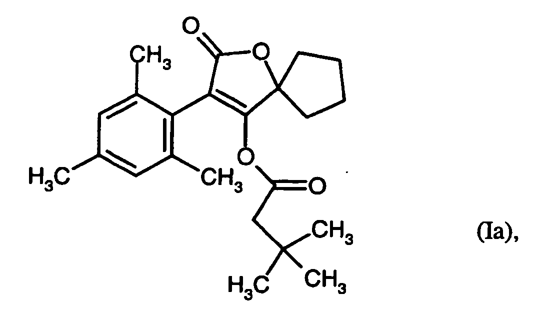 **characterized in that** the compound of the formula (IIa) 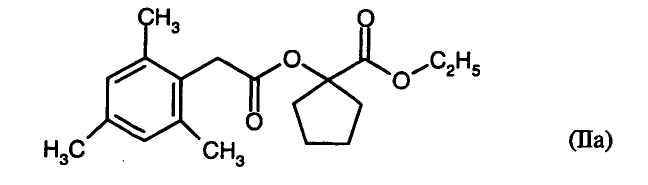 is reacted firstly with NaOH and then with the compound of the formula 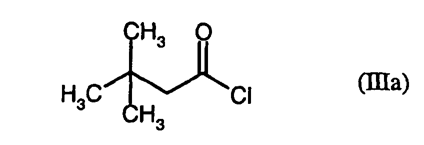 if appropriate in the presence of a diluent.

2. Process for preparing the compound of the formula (Ib) 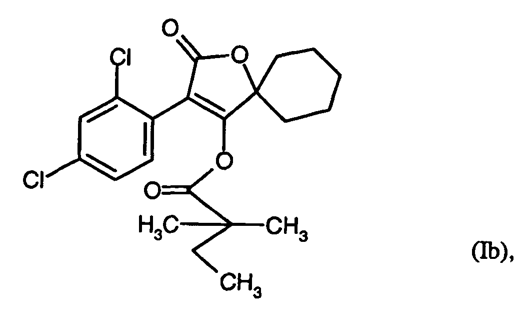 **characterized in that** the compound of the formula (IIb) 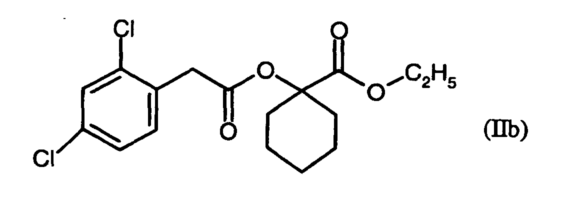 is reacted firstly with NaOH and then with the compound of the formula (IIIb) 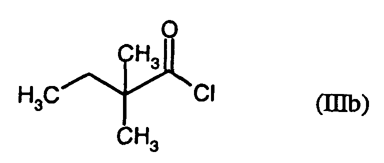 if appropriate in the presence of a diluent.

## Revendications

1. Procédé de production du composé de formule (Ia) 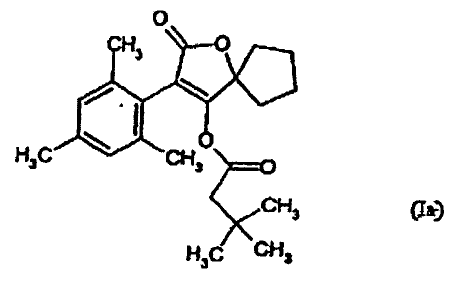 **caractérisé en ce qu'**on fait réagir le composé de formule (IIa) 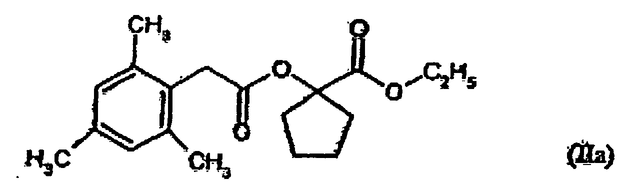 d'abord avec NaOH puis avec le composé de formule 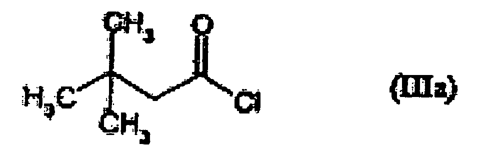 éventuellement en présence d'un diluant.

2. Procédé de production du composé de formule (Ib) 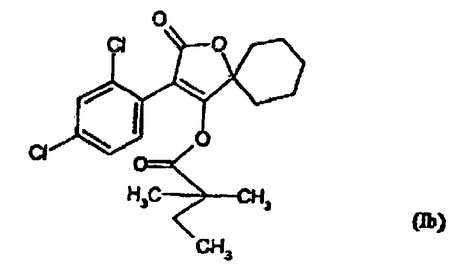 **caractérisé en ce qu'**on fait réagir le composé de formule (IIb) 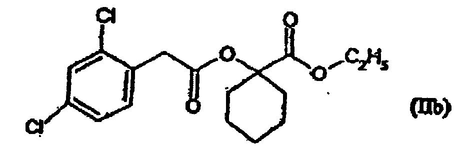 d'abord avec NaOH puis avec le composé de formule (IIIb) 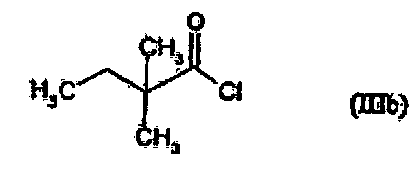 éventuellement en présence d'un diluant.
